# EUROPÄISCHE PATENTANMELDUNG

(11) **EP 2 942 041 A1**
(43) Veröffentlichungstag der Anmeldung: **11.11.2015**
(21) Anmeldenummer: 15166190.7
(22) Anmeldetag: 04.05.2015
(51) Int. Cl.: A61F 7/00, F23Q 2/32

(54) **VORRICHTUNG ZUM BEHANDELN VON INSEKTENSTICHEN**

(30) Priorität: 09.05.2014 DE 202014102158 U
(71) Anmelder: Leithäuser-Rieck, Siegfried, 24229 Strande (DE)
(72) Erfinder: Leithäuser-Rieck, Siegfried, 24229 Strande (DE)
(74) Vertreter: Lobemeier, Martin Landolf

(57) **Zusammenfassung**

Vorrichtung zum Behandeln von Insektenstichen durch kurzzeitiges Erhitzen des betroffenen Bereichs, gekennzeichnet durch eine Manschette (10) zum klemmenden Umgreifen des Körpers eines Feuerzeugs (12), einem von der Manschette (10) abstehenden Hals (14) und einem von dem Hals (14) getragenen, bei Betätigung des Feuerzeugs (12) von dessen Flamme erhitzten Kopf (16).

## Beschreibung

Die Erfindung betrifft eine Vorrichtung zum Behandeln von Insektenstichen durch kurzzeitiges Erhitzen des betroffenen Bereichs.

Aus der DE 199 54 424 A1 ist ein Gerät bekannt, das zur thermischen Behandlung von Insektenstichen dient. Diesem Gerät liegt die Erkenntnis zugrunde, dass thermolabile Insektengifte durch Einbringen einer bestimmten Wärmemenge in den betroffenen Bereich neutralisiert werden können.

Die vorbekannte Einrichtung arbeitet elektrisch, sie bedarf einer Stromversorgung und einer Elektronik.

Der Erfindung liegt die Aufgabe zugrunde, eine Vorrichtung zum Behandeln von Insektenstichen durch kurzzeitiges Erhitzen des betroffenen Bereichs zu schaffen, die einfach aufgebaut und kostengünstig herzustellen ist.

Erfindungsgemäß wird diese Aufgabe gelöst durch eine Vorrichtung, die eine Manschette zum klemmenden Umgreifen des Körpers eines Feuerzeugs, vorzugsweise eines Einwegfeuerzeugs, einen von der Manschette abstehenden Hals und einen von dem Hals getragenen, bei Betätigung des Feuerzeugs von dessen Flamme erhitzten Kopf aufweist. Die Erfindung besteht damit auch in einem Feuerzeug, das die erfindungsgemäße Vorrichtung aufweist.

Bei einer bevorzugten Ausgestaltung besteht der Kopf aus einem sich bei Überschreiten einer bestimmten Temperatur verfärbenden Material oder aber ist auf seiner von der Flamme des Feuerzeugs wegweisenden Fläche mit einem solchen Material belegt.

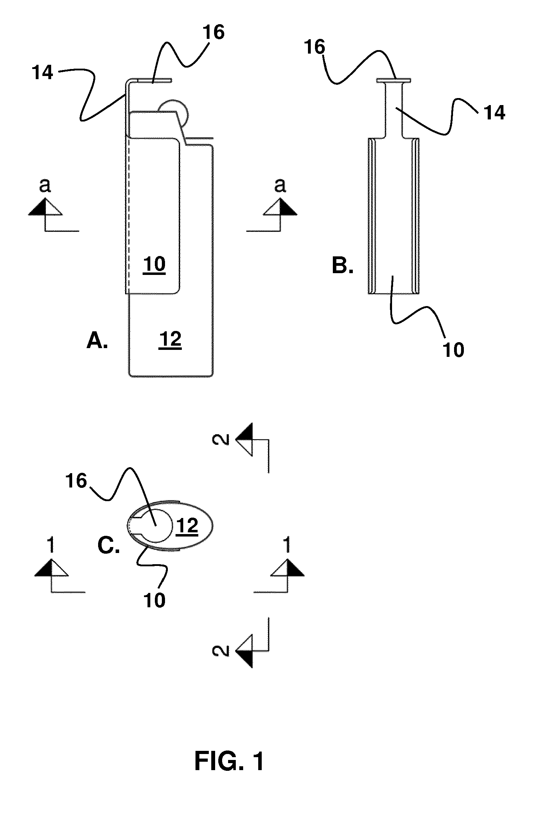

Die Erfindung wird im Folgenden anhand eines in der einzigen Fig. 1 dargestellten, besonders bevorzugten Ausführungsbeispiels näher erläutert, wobei Fig. 1A eine schematische Seitenansicht, Fig. 1B eine Frontalansicht und Fig. 1C eine Schnittansicht entlang der Linie a-a zeigt.

Die Vorrichtung besteht aus einer Manschette 10, die den Körper eines Feuerzeugs 12 klemmend umgreift, wobei das Feuerzeug 12 im Querschnitt oval ausgebildet ist und die Manschette 10 über die Mitte des Feuerzeugs 12 hinübergreift, sodass die Vorrichtung fest auf dem Feuerzeug 12 klemmt.

Von der Manschette 10 steht ein Hals 14 ab, der parallel zu der Achse des Körpers des Feuerzeugs 12 verläuft. Am Ende des Halses 14 ist ein Kopf 16 angesetzt, der bei Aktivierung des Feuerzeugs 12 in oder über dessen Flamme ragt und von der Flamme erhitzt wird. Der Kopf 16 ist bevorzugt rechtwinklig zum Hals 14 angeordnet.

Das Material des Kopfes 16 kann so gewählt sein, dass sich dieses bei Überschreiten einer bestimmten Temperatur, beispielsweise etwa 60 °C, verfärbt. Der Kopf 16 kann alternativ auf seiner von der Flamme des Feuerzeugs 12 weg weisenden Fläche, also in der in Fig. 1A nach oben weisenden Fläche, mit einem solchen Material belegt sein.

Zur Behandlung eines Insektenstiches wird die Vorrichtung auf das Feuerzeug 12 aufgesetzt, das Feuerzeug 12 wird betätigt und der Kopf 16 wird erhitzt (gegebenenfalls bis er durch seine Verfärbung anzeigt, dass er die erforderliche Temperatur erreicht hat).

Sodann wird der Kopf 16 auf den zu behandelnden Bereich des menschlichen Körpers aufgedrückt, ein Teil der Wärmemenge des Kopfes 16 wird unter Erwärmung des betroffenen Bereichs auf diesen übertragen. Die Erwärmung des betroffenen Bereichs führt zu der erwünschten Zersetzung des Insektengiftes.

## Patentansprüche

1. Vorrichtung zum Behandeln eines Insektenstichs durch kurzzeitiges Erhitzen des vom Insektenstich betroffenen Bereichs,
**gekennzeichnet durch**
eine Manschette (10) zum klemmenden Umgreifen des Körpers eines Feuerzeugs (12), einem von der Manschette (10) abstehenden Hals (14) und einem von dem Hals (14) getragenen, bei Betätigung des Feuerzeugs (12) von dessen Flamme erhitzten Kopf (16).

2. Vorrichtung nach Anspruch 1, **dadurch gekennzeichnet, dass** die Manschette (10) zum Umgreifen des im Querschnitt ovalen Körpers des Feuerzeugs (12) geformt ist.

3. Vorrichtung nach einem der vorhergehenden Ansprüche, **dadurch gekennzeichnet, dass** der Kopf (16) aus einem sich bei Überschreiten einer bestimmten Temperatur verfärbenden Material besteht oder auf seiner von der Flamme des Feuerzeugs (12) weg weisenden Fläche mit einem solchen Material belegt ist.

4. Feuerzeug (12) mit einer Vorrichtung nach einem der vorhergehenden Ansprüche.
